# EUROPEAN PATENT SPECIFICATION

(11) **EP 3 975 934 B1**
(45) Date of publication and mention of the grant of the patent: **17.09.2025**
(21) Application number: 20735045.5
(22) Date of filing: 22.05.2020
(51) Int. Cl.: A61F 2/24

(54) **PROSTHETIC HEART VALVE**
HERZKLAPPENPROTHESE
VALVE CARDIAQUE PROTHÉTIQUE

(30) Priority: 29.05.2019 US 201962854244 P
(43) Date of publication of application: 06.04.2022
(73) Proprietor: Edwards Lifesciences Corporation, Irvine, CA 92614 (US)
(72) Inventor: LEVI, Tamir, S., 30889 Caesarea (IL)
(74) Representative: Eisenführ Speiser
(86) International application number: PCT/US2020/034314
(87) International publication number: WO 2020/242980

(56) References cited:
- WO-A1-2018/222799
- US-A1- 2019 053 895

## Description

### FIELD

The present disclosure relates to implantable, expandable prosthetic devices, and in particular, prosthetic heart valves.

### BACKGROUND

The human heart can suffer from various valvular diseases. These valvular diseases can result in significant malfunctioning of the heart and ultimately require replacement of the native valve with an artificial valve. There are a number of known artificial valves and a number of known methods of implanting these artificial valves in humans. Because of the drawbacks associated with conventional open-heart surgery, percutaneous and minimally-invasive surgical approaches are garnering intense attention. In one technique, a prosthetic valve is configured to be implanted in a much less invasive procedure by way of catheterization. For example, collapsible transcatheter prosthetic heart valves can be crimped to a compressed state and percutaneously introduced in the compressed state on a catheter and expanded to a functional size at the desired position by balloon inflation or by utilization of a self-expanding frame or stent. Examples of transcatheter prosthetic heart valves are disclosed in U.S. Patent Nos. 6,730,118, 7,393,360, 7,510,575, and 7,993,394. Further, US 2019/0053895 A1 describes a delivery assembly comprising an annular frame, a leaflet structure positioned within the frame and secured thereto, and an outer skirt positioned around an outer surface of the frame. The annular frame comprises an inflow end and an outflow end and is radially collapsible and expandable between a radially collapsed configuration and a radially expanded configuration. The outer skirt comprises pericardial tissue having a fibrous parietal layer defining a first surface of the outer skirt and a serous parietal layer defining a second surface of the outer skirt. The outer skirt is positioned such that the first surface is facing away from the frame and the second surface is facing towards the frame.

A prosthetic valve for use in such a procedure typically includes a radially collapsible and expandable frame, multiple leaflets supported by the frame, and one or more fabric skirts or cuffs that are used for paravalvular sealing and/or reinforcing the connection between the leaflets and the frame. During the operating cycle of the prosthetic valve, the leaflets open and close under the flow of blood through the valve and may contact the frame and/or a skirt, which may cause abrasion of the leaflets. Thus, it is desirable to prevent or mitigate such abrasion of the leaflets, which can improve long term durability of the prosthetic valve.

### SUMMARY

The present invention relates to an implantable prosthetic valve as defined by appended claim 1. It comprises an annular frame comprising an inflow end and an outflow end and being radially collapsible and expandable between a radially collapsed configuration and a radially expanded configuration; a leaflet (valvular) structure positioned within the frame, the valvular structure being configured to permit the flow of blood from an inflow end to an outflow end of the prosthetic valve and block the flow of blood from the outflow end to the inflow end of the prosthetic valve; and a fabric inner skirt positioned along an inner surface of the frame. The inner skirt comprises a first set of yarns extending in a first direction and a second set of yarns extending in a second direction and woven with the first set of yarns, wherein the yarns of the first set are thinner than the yarns of the second set. The first set of yarns are woven with the second set of yarns such that an inner surface of the skirt is formed from more of the yarns of the first set than the yarns of the second set and an outer surface of the skirt is formed from more of the yarns of the second set than the yarns of the first set.

In some embodiments, the inner surface of the skirt has a coefficient of friction that is less than a coefficient of friction of than the outer surface.

In some embodiments, the inner surface of the skirt is smoother than the outer surface of the skirt.

In some embodiments, the first direction of the first set of yarns extends circumferentially with respect to the frame and the second direction of the second set of yarns extends axially with respect to the frame.

In some embodiments, the yarns of the first and second sets of yarns comprise multi-filament yarns.

In some embodiments, the yarns of the first set have a greater number of filaments than the yarns of the second set.

In some embodiments, the skirt comprises a weave characterized by at least two or more adjacent yarns of the first set floating over a single yarn of the second set and at least two or more yarns of the second set floating over a single yarn of the first set.

In some embodiments, the skirt comprises a weave characterized by at least four or more adjacent yarns of the first set floating over a single yarn of the second set and at least four or more yarns of the second set floating over a single yarn of the first set.

In some embodiments, the yarns of the first set have about 30 to 40 filaments per yarn and the yarns of the second set have about 10 to 20 filaments per yarn.

In some embodiments, the filaments of the first set of yarns have a thickness in a range of about 4 microns to about 6 microns.

In some embodiments, the filaments of the second set of yarns have a thickness in a range of about 10 microns to about 12 microns.

In some embodiments, the skirt comprises a satin weave.

In some embodiments, the leaflet structure comprises a plurality of leaflets having respective inflow edge portions that are sutured to the skirt.

In some embodiments, the skirt is sutured to struts of the frame.

The foregoing and other objects, features, and advantages of the invention will become more apparent from the following detailed description, which proceeds with reference to the accompanying figures.

### BRIEF DESCRIPTION OF THE DRAWINGS

FIGS. 1-3 show an exemplary embodiment of a prosthetic heart valve.
FIGS. 4-10 show an exemplary frame of the prosthetic heart valve of FIG. 1.
FIGS. 11-12 show an exemplary inner skirt of the prosthetic heart valve of FIG. 1.
FIG. 13 shows the prosthetic heart valve of FIG. 1 in a collapsed configuration and mounted on an exemplary balloon catheter.
FIGS. 14-16 show the assembly of the inner skirt of FIG. 11 with the frame of FIG. 4.
FIGS. 17-18 show the assembly of an exemplary leaflet structure.
FIG. 19 shows the assembly of commissure portions of the leaflet structure with window frame portions of the frame.
FIGS. 20-21 show the assembly of the leaflet structure with the inner skirt along a lower edge of the leaflets.
FIG. 22 shows a flattened view of an exemplary outer skirt.
FIG. 23 shows an exemplary embodiment of a weaving pattern for a fabric skirt, as viewed from one side of the fabric.
FIG. 24 shows the weaving pattern of FIG. 23, as viewed from the opposite side of the fabric.
FIG. 25 shows another view of the weaving pattern of FIG. 23.

### DETAILED DESCRIPTION

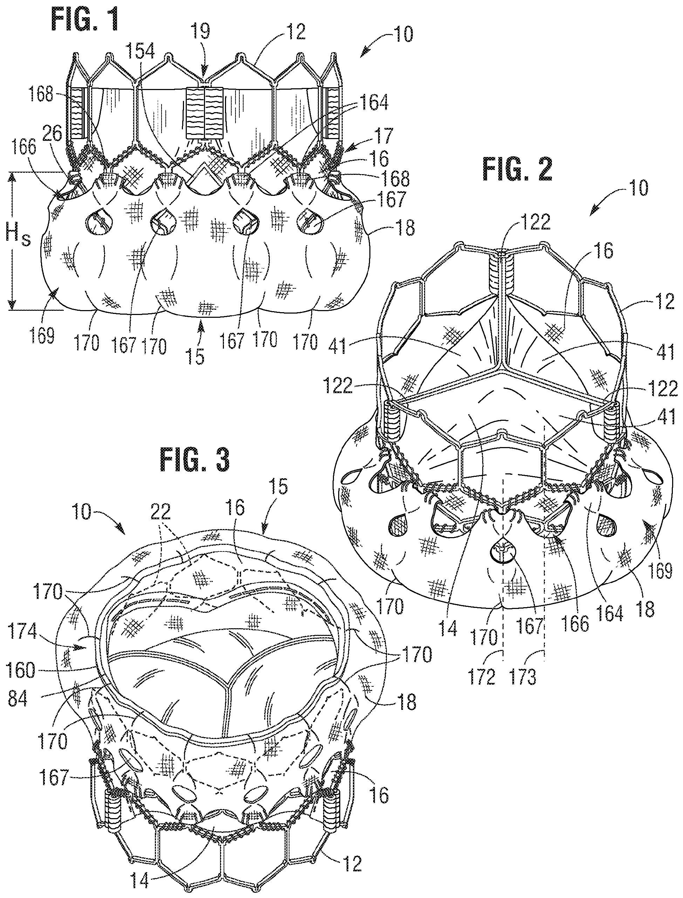

FIGS. 1-3 show various views of a prosthetic heart valve 10, according to one embodiment. The illustrated prosthetic valve is adapted to be implanted in the native aortic annulus, although in other embodiments it can be adapted to be implanted in the other native annuluses of the heart (e.g., the pulmonary, mitral, and tricuspid valves). The prosthetic valve can also be adapted to be implanted in other tubular organs or passageways in the body. The prosthetic valve 10 can have four main components: a stent or frame 12, a valvular structure 14, an inner skirt 16, and a perivalvular sealing means or sealing member. The prosthetic valve 10 can have an inflow end portion 15, an intermediate portion 17, and an outflow end portion 19. In the illustrated embodiment, the perivalvular sealing means comprises an outer skirt 18.

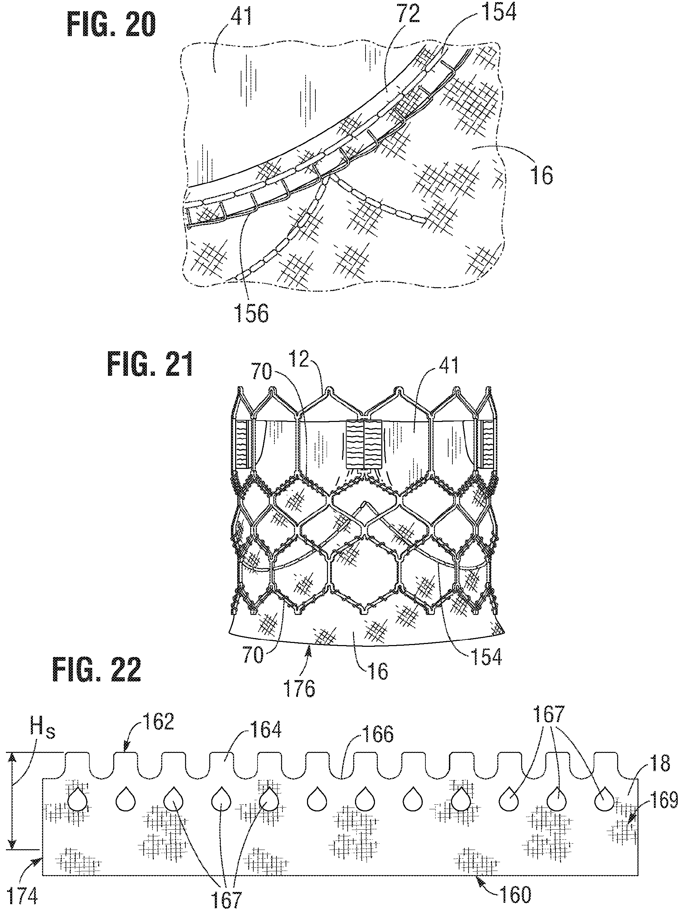

The valvular structure 14 can comprise three leaflets 41, collectively forming a leaflet structure, which can be arranged to collapse in a tricuspid arrangement, as best shown in FIG. 2. The lower edge of leaflet structure 14 desirably has an undulating, curved scalloped shape (suture line 154 shown in FIG. 21 tracks the scalloped shape of the leaflet structure). By forming the leaflets with this scalloped geometry, stresses on the leaflets are reduced, which in turn improves durability of the prosthetic valve. Moreover, by virtue of the scalloped shape, folds and ripples at the belly of each leaflet (the central region of each leaflet), which can cause early calcification in those areas, can be eliminated or at least minimized. The scalloped geometry also reduces the amount of tissue material used to form leaflet structure, thereby allowing a smaller, more even crimped profile at the inflow end of the prosthetic valve. The leaflets 41 can be formed of pericardial tissue (e.g., bovine pericardial tissue), biocompatible synthetic materials, or various other suitable natural or synthetic materials as known in the art and described in U.S. Patent No. 6,730,118.

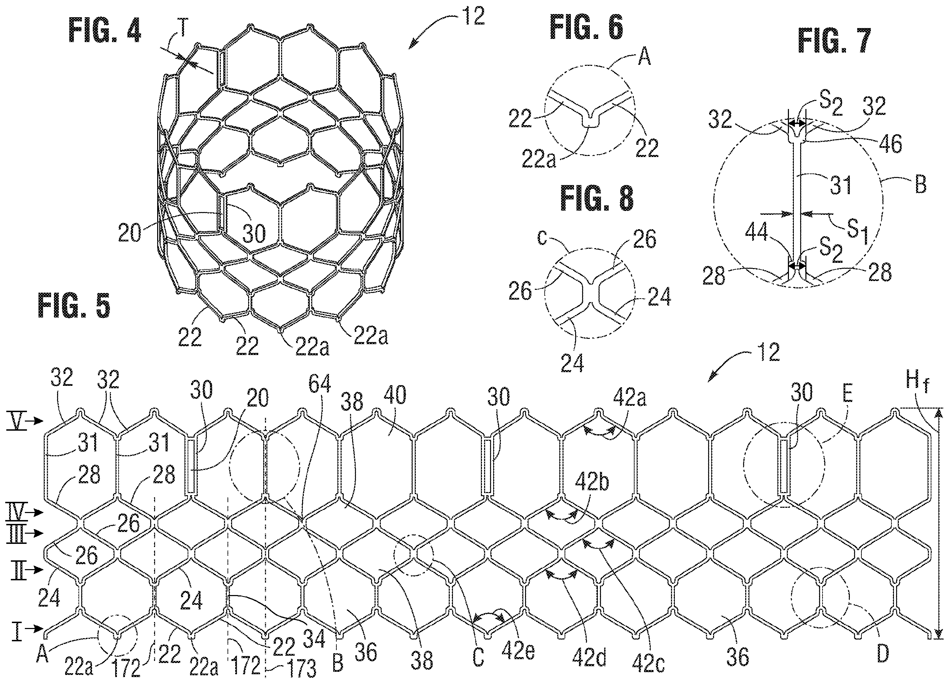

The bare frame 12 is shown in FIG. 4. The frame 12 can be formed with a plurality of circumferentially spaced slots, or commissure windows, 20 (three in the illustrated embodiment) that are adapted to mount the commissures of the valvular structure 14 to the frame, as described in greater detail below. The frame 12 can be made of any of various suitable plastically-expandable materials (e.g., stainless steel, etc.) or self-expanding materials (e.g., nickel titanium alloy (NiTi), such as nitinol) as known in the art. When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular embodiments, frame 12 is made of a nickel-cobalt-chromium-molybdenum alloy, such as MP35N^{®} alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight. It has been found that the use of MP35N^{®} alloy to form frame 12 provides superior structural results over stainless steel. In particular, when MP35N^{®} alloy is used as the frame material, less material is needed to achieve the same or better performance in radial and crush force resistance, fatigue resistances, and corrosion resistance. Moreover, since less material is required, the crimped profile of the frame can be reduced, thereby providing a lower profile prosthetic valve assembly for percutaneous delivery to the treatment location in the body.

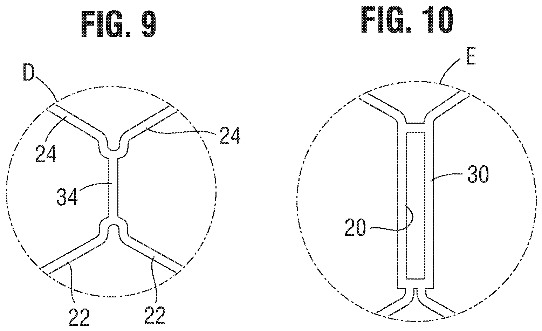

Referring to FIGS. 4 and 5, the frame 12 in the illustrated embodiment comprises a first, lower row I of angled struts 22 arranged end-to-end and extending circumferentially at the inflow end of the frame; a second row II of circumferentially extending, angled struts 24; a third row III of circumferentially extending, angled struts 26; a fourth row IV of circumferentially extending, angled struts 28; and a fifth row V of circumferentially extending, angled struts 32 at the outflow end of the frame. A plurality of substantially straight axially extending struts 34 can be used to interconnect the struts 22 of the first row I with the struts 24 of the second row II. The fifth row V of angled struts 32 are connected to the fourth row IV of angled struts 28 by a plurality of axially extending window frame portions 30 (which define the commissure windows 20) and a plurality of axially extending struts 31. Each axial strut 31 and each frame portion 30 extends from a location defined by the convergence of the lower ends of two angled struts 32 to another location defined by the convergence of the upper ends of two angled struts 28. FIGS. 6, 7, 8, 9, and 10 are enlarged views of the portions of the frame 12 identified by letters A, B, C, D, and E, respectively, in FIG. 5.

Each commissure window frame portion 30 mounts a respective commissure of the leaflet structure 14. As can be seen each frame portion 30 is secured at its upper and lower ends to the adjacent rows of struts to provide a robust configuration that enhances fatigue resistance under cyclic loading of the prosthetic valve compared to known, cantilevered struts for supporting the commissures of the leaflet structure. This configuration enables a reduction in the frame wall thickness to achieve a smaller crimped diameter of the prosthetic valve. In particular embodiments, the thickness T of the frame 12 (FIG. 4) measured between the inner diameter and outer diameter is about 0.48 mm or less.

The struts and frame portions of the frame collectively define a plurality of open cells of the frame. At the inflow end of the frame 12, struts 22, struts 24, and struts 34 define a lower row of cells defining openings 36. The second, third, and fourth rows of struts 24, 26, and 28 define two intermediate rows of cells defining openings 38. The fourth and fifth rows of struts 28 and 32, along with frame portions 30 and struts 31, define an upper row of cells defining openings 40. The openings 40 are relatively large and are sized to allow portions of the leaflet structure 14 to protrude, or bulge, into and/or through the openings 40 when the frame 12 is crimped in order to minimize the crimping profile.

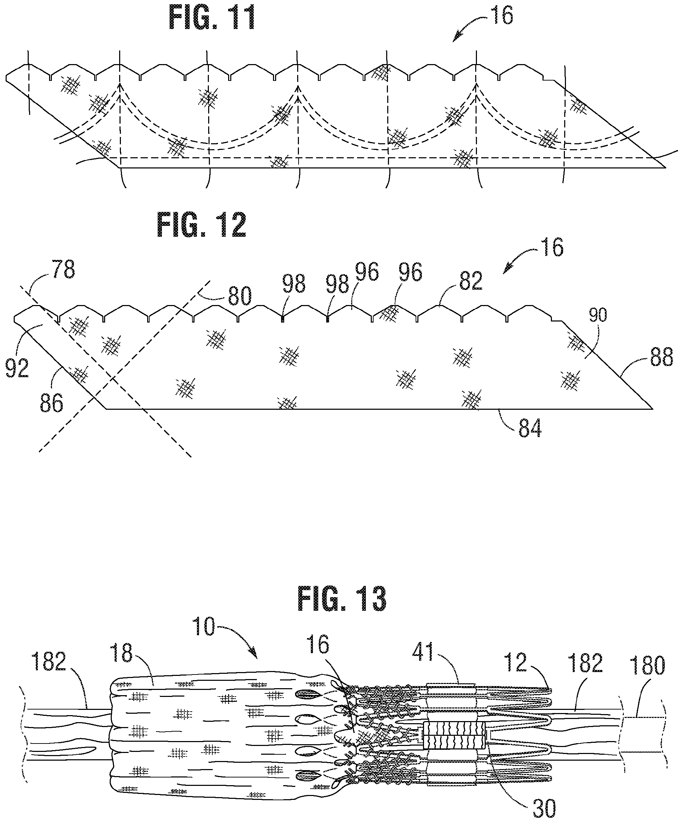

As best shown in FIG. 7, the lower end of the strut 31 is connected to two struts 28 at a node or junction 44, and the upper end of the strut 31 is connected to two struts 32 at a node or junction 46. The strut 31 can have a thickness S1 that is less than the thicknesses S2 of the junctions 44, 46. The junctions 44, 46, along with junctions 64, prevent full closure of openings 40. FIG. 13 shows the prosthetic valve 10 crimped on a balloon catheter. As can be seen, the geometry of the struts 31, and junctions 44, 46, and 64 assists in creating enough space in openings 40 in the collapsed configuration to allow portions of the prosthetic leaflets to protrude or bulge outwardly through openings. This allows the prosthetic valve to be crimped to a relatively smaller diameter than if all of the leaflet material were constrained within the crimped frame.

The frame 12 is configured to reduce, to prevent, or to minimize possible over-expansion of the prosthetic valve at a predetermined balloon pressure, especially at the outflow end portion of the frame, which supports the leaflet structure 14. In one aspect, the frame is configured to have relatively larger angles 42a, 42b, 42c, 42d, 42e between struts, as shown in FIG. 5. The larger the angle, the greater the force required to open (expand) the frame. As such, the angles between the struts of the frame can be selected to limit radial expansion of the frame at a given opening pressure (e.g., inflation pressure of the balloon). In particular embodiments, these angles are at least 110 degrees or greater when the frame is expanded to its functional size, and even more particularly these angles are up to about 120 degrees when the frame is expanded to its functional size.

The main functions of the inner skirt 16 are to assist in securing the valvular structure 14 to the frame 12 and to assist in forming a good seal between the prosthetic valve and the native annulus by blocking the flow of blood through the open cells of the frame 12 below the lower edge of the leaflets. The inner skirt 16 desirably comprises a tough, tear resistant material such as polyethylene terephthalate (PET), although various other synthetic materials or natural materials (e.g., pericardial tissue) can be used. The thickness of the skirt desirably is less than about 0.15 mm (about 6 mil), and desirably less than about 0.1 mm (about 4 mil), and even more desirably about 0.05 mm (about 2 mil). In particular embodiments, the skirt 16 can have a variable thickness, for example, the skirt can be thicker at least one of its edges than at its center. In one implementation, the skirt 16 can comprise a PET skirt having a thickness of about 0.07 mm at its edges and about 0.06 mm at its center. The thinner skirt can provide for better crimping performances while still providing good perivalvular sealing.

The skirt 16 can be secured to the inside of frame 12 via sutures 70, which can extend through the skirt and around struts of the frame, as shown in FIG. 21. Valvular structure 14 can be attached to the skirt via one or more reinforcing strips 72 (which collectively can form a sleeve), for example thin, PET reinforcing strips, discussed below, which enables a secure suturing and protects the pericardial tissue of the leaflet structure from tears. Valvular structure 14 can be sandwiched between skirt 16 and the thin PET strips 72 as shown in FIG. 20. Sutures 154, which secure the PET strip and the leaflet structure 14 to skirt 16, can be any suitable suture, such as Ethibond Excel^{®} PET suture (Johnson & Johnson, New Brunswick, New Jersey). Sutures 154 desirably track the curvature of the inflow or cusp edge (the bottom edge in the illustrated embodiment) of each leaflet structure 41, as described in more detail below.

Known fabric skirts may comprise a weave of warp and weft fibers that extend perpendicularly to each other and with one set of the fibers extending longitudinally between the upper and lower edges of the skirt. When the metal frame to which the fabric skirt is secured is radially compressed, the overall axial length of the frame increases. Unfortunately, a fabric skirt with limited elasticity cannot stretch as the frame elongates and therefore can deform the struts of the frame and prevent uniform crimping unless the skirt includes excess material or slack along its length when in its radially expanded state.

Referring to FIG. 12, in contrast to known fabric skirts, the skirt 16 desirably is woven from a first set of yarns (multi-filament yarns or mono-filament yarns) 78 and a second set of yarns (multi-filament yarns or mono-filament yarns) yarns 80, both of which are non-perpendicular to the upper edge 82 and the lower edge 84 of the skirt. In particular embodiments, the first set of yarns 78 and the second set of yarns 80 extend at angles of about 45 degrees (e.g., 15-75 degrees or 30-60 degrees) relative to the upper and lower edges 82, 84. For example, the skirt 16 can be formed by weaving the yarns at 45-degree angles relative to the upper and lower edges of the fabric. Alternatively, the skirt 16 can be diagonally cut (cut on a bias) from a vertically woven fabric (where the fibers extend perpendicularly to the edges of the material) such that the yarns extend at 45-degree angles relative to the cut upper and lower edges of the skirt. As further shown in FIG. 12, the opposing short edges 86, 88 of the skirt desirably are non-perpendicular to the upper and lower edges 82, 84. For example, the short edges 86, 88 desirably extend at angles of about 45 degrees relative to the upper and lower edges and therefore are aligned with the first set of yarns 78. Therefore, the overall general shape of the skirt is that of a rhomboid or parallelogram.

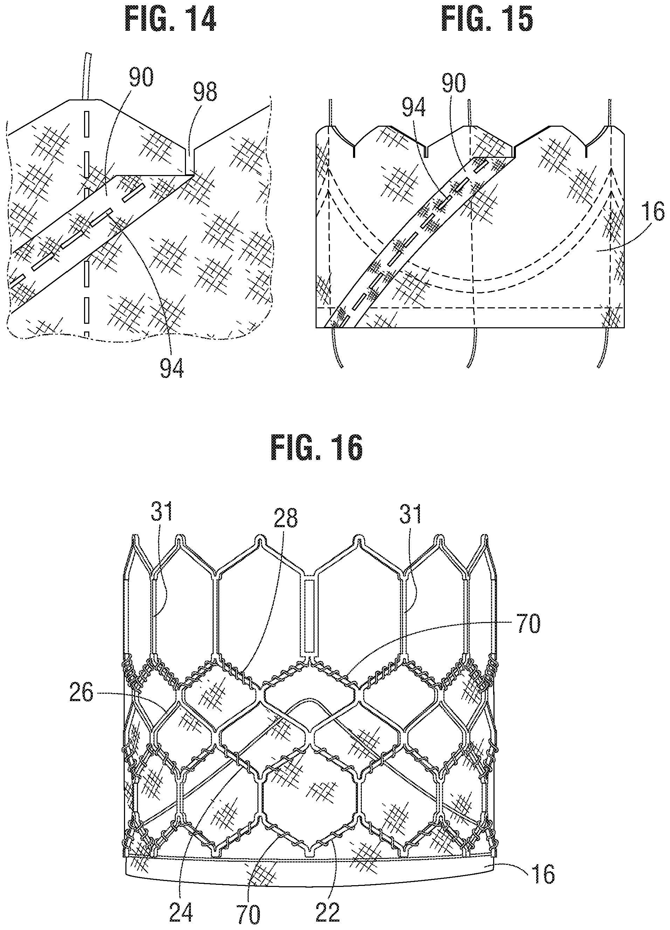

FIGS. 14 and 15 show the inner skirt 16 after opposing short edge portions 90, 92 have been sewn together to form the annular shape of the skirt. As shown, the edge portion 90 can be placed in an overlapping relationship relative to the opposite edge portion 92, and the two edge portions can be sewn together with a diagonally extending suture line 94 that is parallel to short edges 86, 88. The upper edge portion of the inner skirt 16 can be formed with a plurality of projections 96 that define an undulating shape that generally follows the shape or contour of the fourth row of struts 28 immediately adjacent the lower ends of axial struts 31. In this manner, as best shown in FIG. 16, the upper edge of the inner skirt 16 can be tightly secured to struts 28 with sutures 70. The inner skirt 16 can also be formed with slits 98 to facilitate attachment of the skirt to the frame. Slits 98 are dimensioned so as to allow an upper edge portion of the inner skirt 16 to be partially wrapped around struts 28 and to reduce stresses in the skirt during the attachment procedure. For example, in the illustrated embodiment, the inner skirt 16 is placed on the inside of frame 12 and an upper edge portion of the skirt is wrapped around the upper surfaces of struts 28 and secured in place with sutures 70. Wrapping the upper edge portion of the inner skirt 16 around struts 28 in this manner provides for a stronger and more durable attachment of the skirt to the frame. The inner skirt 16 can also be secured to the first, second, and/or third rows of struts 22, 24, and 26, respectively, with sutures 70.

Referring again to FIG. 12, due to the angled orientation of the fibers relative to the upper and lower edges, the skirt can undergo greater elongation in the axial direction (*i.e.,* in a direction from the upper edge 82 to the lower edge 84). Thus, when the metal frame 12 is crimped (as shown in FIG. 14), the inner skirt 16 can elongate in the axial direction along with the frame and therefore provide a more uniform and predictable crimping profile. Each cell of the metal frame in the illustrated embodiment includes at least four angled struts that rotate towards the axial direction on crimping (e.g., the angled struts become more aligned with the length of the frame). The angled struts of each cell function as a mechanism for rotating the fibers of the skirt in the same direction of the struts, allowing the skirt to elongate along the length of the struts. This allows for greater elongation of the skirt and avoids undesirable deformation of the struts when the prosthetic valve is crimped.

In addition, the spacing between the woven yarns can be increased to facilitate elongation of the skirt in the axial direction. For example, for a PET inner skirt 16 formed from 20-denier yarn, the yarn density can be about 15% to about 30% lower than in a typical PET skirt. In some examples, the yarn spacing of the inner skirt 16 can be from about 60 yarns per cm (about 155 yarns per inch) to about 70 yarns per cm (about 180 yarns per inch), such as about 63 yarns per cm (about 160 yarns per inch), whereas in a typical PET skirt the yarn spacing can be from about 85 yarns per cm (about 217 yarns per inch) to about 97 yarns per cm (about 247 yarns per inch). The oblique edges 86, 88 promote a uniform and even distribution of the fabric material along inner circumference of the frame during crimping so as to reduce or minimize bunching of the fabric to facilitate uniform crimping to the smallest possible diameter. Additionally, cutting diagonal sutures in a vertical manner may leave loose fringes along the cut edges. The oblique edges 86, 88 help minimize this from occurring. Compared to the construction of a conventional skirt, the construction of the inner skirt 16 avoids undesirable deformation of the frame struts and provides more uniform crimping of the frame.

In alternative embodiments, the skirt can be formed from woven elastic yarns that can stretch in the axial direction during crimping of the prosthetic valve. The warp and weft yarns can run perpendicularly and parallel to the upper and lower edges of the skirt, or alternatively, they can extend at angles between 0 and 90 degrees relative to the upper and lower edges of the skirt, as described above.

The inner skirt 16 can be sutured to the frame 12 at locations away from the suture line 154 so that the skirt can be more pliable in that area. This configuration can avoid stress concentrations at the suture line 154, which attaches the lower edges of the leaflets to the inner skirt 16.

As noted above, the leaflet structure 14 in the illustrated embodiment includes three flexible leaflets 41 (although a greater or a smaller number of leaflets can be used). Additional information regarding the leaflets, as well as additional information regarding skirt material, can be found, for example, in U.S. Patent No. 10,195,025.

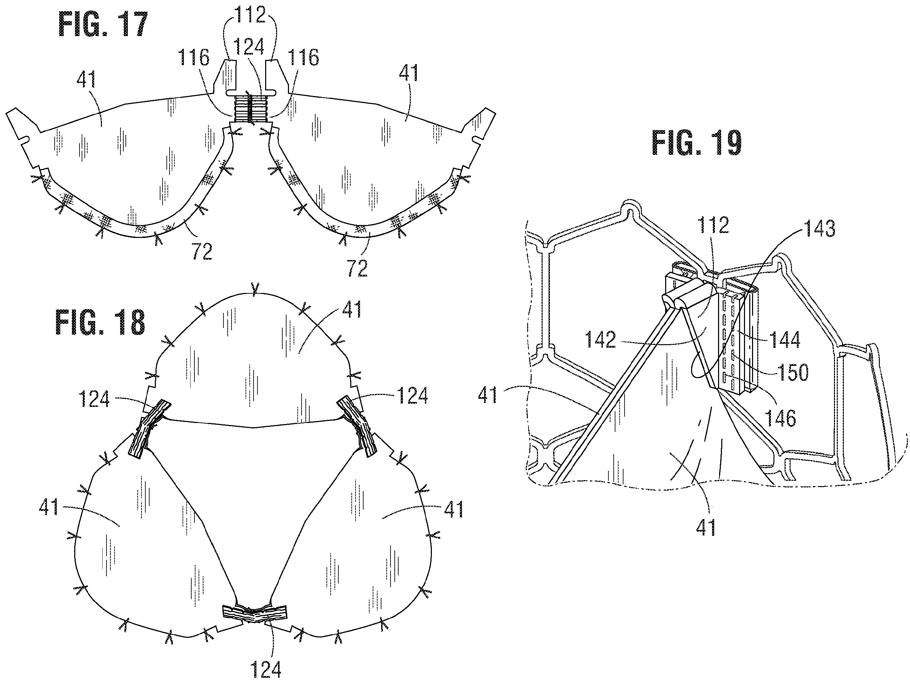

The leaflets 41 can be secured to one another at their adjacent sides to form commissures 122 of the leaflet structure. A plurality of flexible connectors 124 (one of which is shown in FIG. 17) can be used to interconnect pairs of adjacent sides of the leaflets and to mount the leaflets to the commissure window frame portions 30 (FIG. 5).

FIG. 17 shows the adjacent sides of two leaflets 41 interconnected by a flexible connector 124. Three leaflets 41 can be secured to each other side-to-side using three flexible connectors 124, as shown in FIG. 18. Additional information regarding connecting the leaflets to each other, as well as connecting the leaflets to the frame, can be found, for example, in U.S. Patent Application Publication No. 2012/0123529.

As noted above, the inner skirt 16 can be used to assist in suturing the leaflet structure 14 to the frame. The inner skirt 16 can have an undulating temporary marking suture to guide the attachment of the lower edges of each leaflet 41. The inner skirt 16 itself can be sutured to the struts of the frame 12 using sutures 70, as noted above, before securing the leaflet structure 14 to the skirt 16. The struts that intersect the marking suture desirably are not attached to the inner skirt 16. This allows the inner skirt 16 to be more pliable in the areas not secured to the frame and minimizes stress concentrations along the suture line that secures the lower edges of the leaflets to the skirt. As noted above, when the skirt is secured to the frame, the yarns 78, 80 of the skirt (see FIG. 12) generally align with the angled struts of the frame to promote uniform crimping and expansion of the frame.

FIG. 19 shows one specific approach for securing the commissure portions 122 of the leaflet structure 14 to the commissure window frame portions 30 of the frame. The flexible connector 124 (FIG. 18) securing two adjacent sides of two leaflets is folded widthwise and respective upper tab portions 112 of the leaflets are folded downwardly against the flexible connector. Each upper tab portion 112 is creased lengthwise (vertically) to assume an L-shape having an inner portion 142 folded against the inner surface of the leaflet and an outer portion 144 folded against the connector 124. The outer portion 144 can then be sutured to the connector 124 along a suture line 146. Next, the commissure tab assembly is inserted through the commissure window 20 of a corresponding window frame portion 30, and the folds outside of the window frame portion 30 can be sutured to portions 144.

FIG. 19 also shows that the folded down upper tab portions 112 can form a double layer of leaflet material at the commissures. The inner portions 142 of the upper tab portions 112 are positioned flat against layers of the two leaflets 41 forming the commissures, such that each commissure comprises four layers of leaflet material just inside of the window frames 30. This four-layered portion of the commissures can be more resistant to bending, or articulating, than the portion of the leaflets 41 just radially inward from the relatively more-rigid four-layered portion. This causes the leaflets 41 to articulate primarily at inner edges 143 of the folded-down inner portions 142 in response to blood flowing through the prosthetic valve during operation within the body, as opposed to articulating about or proximal to the axial struts of the window frames 30. Because the leaflets articulate at a location spaced radially inwardly from the window frames 30, the leaflets can avoid contact with and damage from the frame. However, under high forces, the four layered portion of the commissures can splay apart about a longitudinal axis adjacent to the window frame 30, with each inner portion 142 folding out against the respective outer portion 144. For example, this can occur when the prosthetic valve 10 is compressed and mounted onto a delivery shaft, allowing for a smaller crimped diameter. The four-layered portion of the commissures can also splay apart about the longitudinal axis when the balloon catheter is inflated during expansion of the prosthetic valve, which can relieve some of the pressure on the commissures caused by the balloon, reducing potential damage to the commissures during expansion.

After all three commissure tab assemblies are secured to respective window frame portions 30, the inflow or cusp edges of the leaflets 41 (the lower edges in the illustrated embodiment) between the commissure tab assemblies can be sutured to the inner skirt 16. For example, as shown in FIG. 20, each leaflet 41 can be sutured to the inner skirt 16 along suture line 154 using, for example, Ethibond Excel^{®} PET thread. The sutures can be in-and-out sutures extending through each leaflet 41, the inner skirt 16, and each reinforcing strip 72. Each leaflet 41 and respective reinforcing strip 72 can be sewn separately to the inner skirt 16. In this manner, the lower edges of the leaflets are secured to the frame 12 via the inner skirt 16. As shown in FIG. 20, the leaflets can be further secured to the skirt with blanket sutures 156 that extend through each reinforcing strip 72, leaflet 41 and the inner skirt 16 while looping around the edges of the reinforcing strips 72 and leaflets 41. The blanket sutures 156 can be formed from PTFE suture material. FIG. 21 shows a side view of the frame 12, leaflet structure 14 and the inner skirt 16 after securing the leaflet structure 14 and the inner skirt 16 to the frame 12 and the leaflet structure 14 to the inner skirt 16.

FIG. 22 shows a flattened view of the outer skirt 18 prior to its attachment to the frame 12. The outer skirt 18 can be laser cut or otherwise formed from a strong, durable material such as PET or various other suitable synthetic or natural materials configured to restrict and/or prevent blood-flow therethrough. The outer skirt 18 can comprise a substantially straight lower edge 160 and an upper edge portion 162 defining a plurality of alternating projections 164 and notches 166, or castellations. The outer skirt 18 can also comprise a plurality of openings 167 (e.g., 12 in the illustrated embodiment) disposed on an intermediate portion 169 (i.e., the portion between the lower edge 160 and the upper edge portion 162) of the outer skirt 18. The openings 167 are spaced from the lower edge 160 and the upper edge portion 162 such that the material of the outer skirt 18 separates the openings 167 from the lower edge 160 and the upper edge portion 162.

As best shown in FIG. 3, a lower edge portion 174 of the outer skirt 18 can be wrapped around the inflow end 15 of the frame 12, and the lower edge 160 of the outer skirt 18 can be attached to the lower edge 84 of the inner skirt 16 and/or the frame 12 at the inflow end of the prosthetic valve 10. In some embodiments, the outer skirt 18 can be attached to the inner skirt 16, for example, with sutures or a suitable adhesive.

In lieu of or in addition to sutures, the outer skirt 18 can be attached to the inner skirt 16, for example, by ultrasonic welding. Ultrasonic welding can provide several significant advantages. For example, ultrasonic welding can be relatively less time consuming and less expensive compared to suturing, while also providing improved strength.

As shown in FIG. 1, each projection 164 of the outer skirt 18 can be attached to the third row III of struts 26 (FIG. 5) of the frame 12. The projections 164 can, for example, be wrapped over respective struts 26 of row III and secured with sutures 168.

As can be seen in FIGS. 1-3, the outer skirt 18 is secured to the frame 12 such that when the frame is in its expanded configuration (e.g., when deployed in a subject), there is excess material between the lower edge 160 and the upper edge portion 162 that does not lie flat against the outer surface of the frame 12. The outer skirt 18 can be secured directly to frame 12 and/or indirectly to frame 12, for example, by securing the outer skirt 18 to the inner skirt 16, which is directly secured to the frame 12. In the expanded configuration of the prosthetic valve, the distance between the upper and lower attachment points of the outer skirt 18 decreases (foreshortens), resulting in radial expansion of the outer skirt 18. Additionally, the excess material between the lower and upper edges of the outer skirt 18 allows the frame 12 to elongate axially when crimped without any resistance from the outer skirt 18.

The outer skirt 18 can comprise an axial length or height Hₛ, where Hₛ is the height of the outer skirt 18, less the lower edge portion 174 that is wrapped around the inflow end 15 of the frame 12, as best shown in FIGS. 1, 3, and 22. In some embodiments, the height Hₛ can be substantially the same as the axial length between the upper attachment point of the outer skirt 18 to the frame 12 and the inflow end 15 of the frame 12 when the frame 12 is fully crimped. In such embodiments, when the frame 12 is fully crimped, the outer skirt 18 can lie flat against the outer surface of the frame 12. In other embodiments, the height Hₛ of the outer skirt 18 can exceed the axial length between the upper attachment point of the outer skirt 18 to the frame 12 and the inflow end 15 of the frame 12 when the frame 12 is fully crimped. In such embodiments, the outer skirt 18 can comprise a plurality of creases 170 (e.g., twelve in the illustrated embodiment).

As best shown in FIG. 3, the creases 170 can extend axially from the lower edge 160 toward the intermediate portion 169 of the outer skirt 18. The creases 170 can be aligned circumferentially with respective projections 164, and the outer skirt 18 can be oriented with respect to the frame 12 such that the creases 170 are circumferentially aligned between a respective pair of apices 22a (FIG. 5) that are formed by the struts 22 at the inflow end 15 of the frame 12. For example, the creases 170 can be circumferentially aligned along a respective vertical line 172 (FIGS. 2 and 5) that is parallel to the longitudinal axis of the frame 12 and bisects the frame 12 at a location equidistant from each apex 22a of a respective pair of apices 22a. In this manner, the creases 170 can cause excess material of the outer skirt 18 to retract radially inwardly between the apices 22a and into the inflow end 15 of the frame 12 when the prosthetic valve 10 is crimped from the expanded configuration. As best shown in FIG. 2, each crease 170 can be circumferentially aligned with a respective opening 167 and projection 164 along a respective line 172.

As best shown in FIG. 22, the openings 167 can be laterally (circumferentially in FIGS. 1-3) spaced apart relative to adjacent openings 167 and be laterally (circumferentially in FIGS. 1-3) aligned with a respective projection 164. The openings 167 can also be circumferentially aligned with respective creases 170, as best shown in FIGS. 1 and 3. For example, the projections 164, the openings 167, and the creases 170 can be aligned along the respective vertical lines 172, as best shown in FIG. 2. Aligning the openings 167 and the creases 170 can, for example, allow blood to quickly enter, and thus expand, the overlapped portions of the outer skirt 18 when the prosthetic valve is initially deployed, as further described below.

The openings 167 can also advantageously allow back-flowing blood (e.g., retrograde blood) to enter the outer skirt 18 from a different angle or direction than the notches 166, thus improving how quickly the outer skirt 18 initially expands and improving perivalvular sealing. The openings 167 can be provided in lieu of or in addition to the notches 166.

The openings 167 can comprise various shapes. For example, the openings 167 can comprise a tear-drop shape, as shown in the illustrated embodiment. In other embodiments, the openings can be circular, elliptical, rectangular, etc.

The prosthetic valve 10 can be configured for and mounted on a suitable delivery apparatus for implantation in a subject. Several catheter-based delivery apparatuses are known; a non-limiting example of a suitable catheter-based delivery apparatus includes those disclosed in U.S. Patent Application Publication Nos. 2013/0030519 and 2017/0065415, and U.S. Patent Application Publication No. 2012/0123529.

To implant a plastically-expandable prosthetic valve 10 within a patient, the prosthetic valve 10 can be crimped on an elongated shaft 180 of a delivery apparatus, as best shown in FIG. 13. The prosthetic valve, together with the delivery apparatus, can form a delivery assembly for implanting the prosthetic valve 10 in a patient's body. The shaft 180 comprises an inflatable balloon 182 for expanding the prosthetic valve within the body. With the balloon 182 deflated and the prosthetic valve crimped around the balloon, the prosthetic valve 10 can then be percutaneously delivered to a desired implantation location (e.g., a native aortic valve region). Once the prosthetic valve 10 is delivered to the implantation site (e.g., the native aortic valve) inside the body, the prosthetic valve 10 can be radially expanded to its functional state by inflating the balloon 182.

When the prosthetic valve 10 expands, the notches 166 and the openings 167 allow blood to flow between the outer skirt 18 and the inner skirt 16. This blood-flow causes the excess fabric of the outer skirt 18 to further radially expand and separate from the inner skirt 16.

The expanded outer skirt 18 can fill-in gaps between the frame 12 and the surrounding native annulus to assist in forming a good, fluid-tight seal between the prosthetic valve 10 and the native annulus. The outer skirt 18 therefore cooperates with the inner skirt 16 to avoid perivalvular leakage after implantation of the prosthetic valve 10. In several embodiments, the prosthetic valve 10 comprising the outer skirt 18 that expands radially outwardly can have reduced perivalvular leakage when implanted in a subject compared to a similar prosthetic valve that has a relatively smaller outer skirt or lacks the outer skirt 18.

Alternatively, a self-expanding prosthetic valve 10 can be crimped to a radially collapsed configuration and restrained in the collapsed configuration by inserting the prosthetic valve 10 into a sheath or equivalent mechanism of a delivery catheter. The prosthetic valve 10 can then be percutaneously delivered to a desired implantation location. Once inside the body, the prosthetic valve 10 can be advanced from the delivery sheath, which allows the prosthetic valve to expand to its functional state.

When the outer skirt 18 is exposed from the delivery sheath, the notches 166 and the openings 167 allow blood to flow between the outer skirt 18 and the inner skirt 16. This blood-flow causes the excess fabric of the outer skirt 18 to further radially expand and separate from the inner skirt 16.

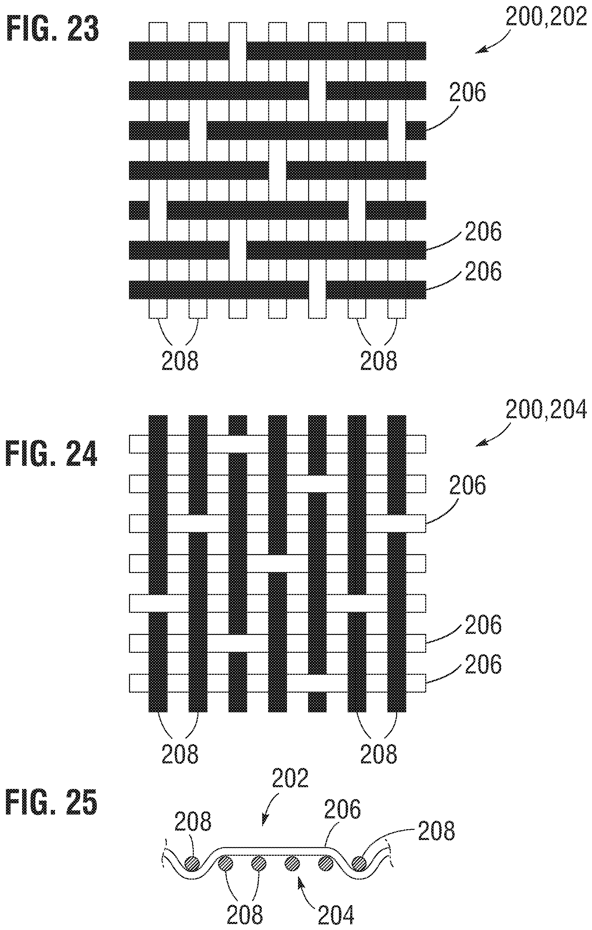

FIGS. 23-25 shows an exemplary embodiment of a weaving pattern for forming a fabric skirt 200. FIG. 23 shows one surface of the skirt 200 and FIG. 24 shows the opposite surface of the skirt 200. The skirt 200 may be used as an inner skirt and/or an outer skirt of a prosthetic valve 10. In particular embodiments, the skirt 200 is an inner skirt of a prosthetic valve 10, and FIG. 23 represents the inner surface 202 of the inner skirt (the surface facing the leaflets 41) and FIG. 24 represents the outer surface 204 of the inner skirt (the surface facing toward from the frame 12). The skirt 200 can have the same overall size and shape of the inner skirt 16 described above and can be assembled to the frame 12 and the leaflets 41 as previously described.

The skirt 200 comprises a first set of yarns 206 woven together with a second set of yarns 208. The yarns 206, 208 can comprise multi-filament yarns (yarns comprising plural fibers or filaments) or mono-filament yarns (yarns comprising single fibers or filaments). In the illustrated embodiment, the yarns 206 are oriented in the circumferential direction of the prosthetic valve 10 and the yarns 208 are oriented in the axial direction of the prosthetic valve. During manufacture of the skirt 200, the yarns 206 can be the weft yarns of the fabric and the yarns 208 can be the warp yarns of the fabric. However, in alternative embodiments, the yarns 206 can be the warp yarns of the fabric and the yarns 208 can be the weft yarns of the fabric.

Due to the weave of the fabric, the inner surface 202 of the skirt is formed from more of yarns 206 than the yarns 208, while the outer surface 204 of the skirt is formed from more of the yarns 208 than the yarns 206. Stated differently, more of the yarns 206 are exposed on the inner surface 202 than the yarns 208, while more of the yarns 208 are exposed on the outer surface 204 than the yarns 206. Specifically, as best shown in FIG. 25, in the illustrated embodiment, each yarn 206 extends under one yarn 208 on the outer surface 204, floats over four yarns 208 on the inner surface 202, then extends under one yarn 208 on the outer surface 204, and repeats this pattern along the length of the yarn. Thus, along the length of each yarn 208 in the axial direction of the surface 202 (vertically in FIG. 23) there are four adjacent yarns 206 that float over the same yarn 208 between two interlacings of that yarn 208 and the yarns 206. Similarly, each yarn 208 extends over one yarn 206 on the inner surface 202, floats over four yarns 206 on the outer surface 204, then extends over one yarn 206 on the inner surface 202, and repeats this pattern along the length of the yarn. Thus, along the length of each yarn 206 in the circumferential direction of the surface 204 (horizontally in FIG. 24) there are four adjacent yarns 208 that float over the same yarn 206 between two interlacings of that yarn 206 and the yarns 208. In this manner, the yarns 206 dominate the inner surface 202 of the fabric and the yarns 208 dominate the outer surface 204 of the fabric. Stated differently, the majority of the surface area of the inner surface 202 comprises yarns 206 and the majority of the surface area of the outer surface 204 comprises yarns 208.

Although the illustrated embodiment is characterized by four floats between interlacings (where a yarn extends from one surface of the fabric to the opposite surface of the fabric), the number of floats for the yarns 206, 208 can vary. In particular embodiments, each yarn 206, 208 has at least two floats between interlacings, at least three floats between interlacings, at least four floats between interlacings, at least five floats between interlacings, at least six floats between interlacings, at least seven floats between interlacings, at least eight floats between interlacings, etc. In certain embodiments, the number of floats between interlacings can vary along the length of a yarn and/or among the yarns.

In particular embodiments, the yarns 206, 208 comprises PET, although other biocompatible synthetic or natural fibers may be used.

The fabric shown in FIGS. 23-25 may be referred to a satin fabric or a fabric having a satin weave. As used herein, the term "satin" refers to a weave or fabric having a first set of yarns interwoven with a second set of yarns with at least two floats between interlacings such that the first set of yarns dominate one surface of the fabric and the second set of yarns dominate the other surface of the fabric. In alternative embodiments, other weaving patterns having a first set of yarns dominating one surface and a second set of yarns dominating another surface may be employed, such as a twill or a herringbone weaving pattern.

In particular embodiments, the number of yarns 206 are greater than the number of yarns 208. Also, in particular embodiments, the yarns 206 have a diameter or thickness that is smaller than the diameter or thickness of the yarns 208. Due to the dominance of the relatively thinner yarns 206 on the inner surface 202, the inner surface 202 is relatively smoother and less rough than the outer surface 204. Moreover, the inner surface 202 has a lower coefficient of friction than the outer surface 204. Advantageously, the inner surface 204 can reduce abrasion of the leaflets 41 if they contact the inner skirt as the leaflets 41 open during working cycles of the prosthetic valve. In addition, the relatively thinner yarns 206 can reduce creasing and fold lines in the skirt after the prosthetic valve is radially expanded to its functional size.

As noted above, components 206, 208 can represent interwoven first and second yarns 206, 208, each formed from multiple filaments or fibers. In particular embodiments, the yarns 206 each comprise multiple filaments that have a diameter or thickness that is smaller than the diameter or thickness of the filaments of the yarns 208. Also, in the particular embodiments, the yarns 206 have a greater number of filaments per yarn than the yarns 208. Due to the dominance of the yarns 206 of relatively thinner filaments on the inner surface 202, the inner surface 202 is relatively smoother and less rough and has a lower coefficient of friction than the outer surface 204.

In certain embodiments, the yarns 206 have about 20 to 50 filaments per yarn, more desirably about 25 to 45 filaments per yarn, even more desirably about 30 to 40 filaments per yarn, and even more desirably about 30 to 35 filaments per yarn, with 32 filaments per yarn being a specific example. The filaments of the yarns 206 can have a thickness in the range of about 2 microns to about 10 microns, more desirably about 4 microns to about 6 microns, with 5 microns being a specific example. The yarns 208 can have about 15 to 25 filaments per yarn, more desirably 10 to 20 filaments per yarn, even more desirably about 15 to 20 filaments per yarn, with 18 filaments per yarn being a specific example. The filaments of the yarns 208 can have a thickness in the range of about 8 microns to about 16 microns, more desirably about 10 microns to about 12 microns, with 11 microns being a specific example.

As noted above, the relatively thicker yarns 208 dominate the outer surface 204 of the skirt 200 can be oriented to extend axially along the frame 12 of the prosthetic valve. In some embodiments, the outer surface 204 of the skirt can come into contact with an inner surface of an introducer sheath through the openings in the frame 12. The axially oriented yarns 208 can reduce sliding friction with the introducer sheath and can reduce the pushing force required to advance the prosthetic valve through the introducer sheath. Similarly, in some embodiments the outer surface 204 of the skirt can come into contact with a delivery capsule or sheath (such as in the case of a self-expandable prosthetic valve). The axially oriented yarns 208 can reduce sliding friction with the delivery capsule and can reduce the pushing or pulling force required to deploy the prosthetic valve from the delivery capsule.

In alternative embodiments, the yarns 206 can be oriented axially and the yarns 208 can be oriented circumferentially. In still alternative embodiments, the yarns 206, 208 can be non-perpendicular to the inflow and outflow edges of the skirt, such as described above with respect to the skirt 16 shown in FIGS. 11 and 12 to facilitate radial crimping of the prosthetic valve. For example, the yarns 206, 208 can be oriented at angles of about 15-75 degrees with respect to the inflow and outflow edges, or more desirably about 30-60 degrees with respect to the inflow and outflow edges, with 45 degrees being a specific example.

In alternative embodiments, it may be desirable to reverse the orientation of the inner skirt 200 such that the surface 202 is an outer surface facing the frame 12 and the surface 204 is an inner surface facing inwardly toward the leaflets 41, for example, if the leaflets 41 do not come into contact with the skirt during working cycles of the prosthetic valve.

In alternative embodiments, the yarns 208 can have the same number of filaments and/or filaments of the same thickness as the filaments of the yarns 206, providing a fabric wherein both surfaces 202, 204 are relatively smooth. For example, in certain implementations, the yarns 206, 208 have about 20 to 50 filaments per yarn, more desirably about 25 to 45 filaments per yarn, even more desirably about 30 to 40 filaments per yarn, and even more desirably about 30 to 35 filaments per yarn, with 32 filaments per yarn being a specific example. The filaments of the yarns 206, 208 can have a thickness in the range of about 2 microns to about 10 microns, more desirably about 4 microns to about 6 microns, with 5 microns being a specific example.

In another embodiment, the skirt 200 can be an outer skirt of a prosthetic valve. The outer skirt 200 can have an inner surface 202 facing the frame 12 and an outer surface 204 facing away from the frame 12. This may be desirable in embodiments of the prosthetic valve where an inner skirt is not provided, and the outer skirt can come into contact with the leaflets through the cells of the frame. In other implementations, the orientation of the outer skirt 200 can be reversed such that the surface 202 is the outer surface of the outer skirt and the surface 204 is the inner surface of the outer skirt outer skirt. In one specific example, the prosthetic valve 10 can have an outer skirt 200 that can have the same overall shape and configuration as the outer skirt 18 and can be assembled in the same manner as the outer skirt 18.

### General Considerations

It should be understood that the disclosed embodiments can be adapted to deliver and implant prosthetic devices in any of the native annuluses of the heart (e.g., the aortic, pulmonary, mitral, and tricuspid annuluses), and can be used with any of various approaches (e.g., retrograde, antegrade, transseptal, transventricular, transatrial, etc.).

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the terms "coupled" and "associated" generally mean electrically, electromagnetically, and/or physically (e.g., mechanically or chemically) coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device toward the user, while distal motion of the device is motion of the device away from the user. The terms "longitudinal" and "axial" refer to an axis extending in the proximal and distal directions, unless otherwise expressly defined.

As used herein, the terms "integrally formed" and "unitary construction" refer to a construction that does not include any welds, fasteners, or other means for securing separately formed pieces of material to each other.

As used herein, the term "coupled" generally means physically coupled or linked and does not exclude the presence of intermediate elements between the coupled items absent specific contrary language.

As used herein, operations that occur "simultaneously" or "concurrently" occur generally at the same time as one another, although delays in the occurrence of one operation relative to the other due to, for example, spacing, play or backlash between components in a mechanical linkage such as threads, gears, etc., are expressly within the scope of the above terms, absent specific contrary language.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims.

## Claims

1. An implantable prosthetic valve (10) comprising:
an annular frame (12) comprising an inflow end and an outflow end and being radially collapsible and expandable between a radially collapsed configuration and a radially expanded configuration;
a leaflet structure (14) positioned within the frame (12), the leaflet structure (14) being configured to permit the flow of blood from an inflow end to an outflow end of the prosthetic valve (10) and block the flow of blood from the outflow end to the inflow end of the prosthetic valve (10); and
a fabric inner skirt (16; 200) positioned along an inner surface of the frame (12), wherein the inner skirt (16; 200) comprises a first set of yarns (78) extending in a first direction and a second set of yarns (80) extending in a second direction and woven with the first set of yarns (78), wherein the yarns of the first set (78) are thinner than the yarns of the second set (80);
wherein the first set of yarns (78) are woven with the second set of yarns (80) such that an inner surface (202) of the skirt (16; 200) is formed from more of the yarns of the first set (78) than the yarns of the second set (80) and an outer surface (204) of the skirt (16; 200) is formed from more of the yarns of the second set (80) than the yarns of the first set (78).

2. The prosthetic valve (10) of claim 1, wherein the inner surface (202) of the skirt (16; 200) has a coefficient of friction that is less than a coefficient of friction of than the outer surface (204).

3. The prosthetic valve (10) of any previous claim, wherein the inner surface (202) of the skirt (16; 200) is smoother than the outer surface (204) of the skirt (16; 200).

4. The prosthetic valve (10) of any previous claim, wherein the first direction of the first set of yarns (78) extends circumferentially with respect to the frame (12) and the second direction of the second set of yarns (80) extends axially with respect to the frame (12).

5. The prosthetic valve (10) of any previous claim, wherein the yarns of the first and second sets of yarns (78, 80) comprise multi-filament yarns.

6. The prosthetic valve (10) of claim 5, wherein the yarns of the first set (78) have a greater number of filaments than the yarns of the second set (80).

7. The prosthetic valve (10) of any previous claim, wherein the skirt (16; 200) comprises a weave **characterized by** at least two or more adjacent yarns of the first set (78) floating over a single yarn of the second set (80) and at least two or more yarns of the second set (80) floating over a single yarn of the first set (78).

8. The prosthetic valve (10) of claim 7, wherein the skirt (16; 200) comprises a weave **characterized by** at least four or more adjacent yarns of the first set (78) floating over a single yarn of the second set (80) and at least four or more yarns of the second set (80) floating over a single yarn of the first set (78).

9. The prosthetic valve (10) of claim 5, wherein the yarns of the first set (78) have about 30 to 40 filaments per yarn and the yarns of the second set (80) have about 10 to 20 filaments per yarn.

10. The prosthetic valve (10) of claim 5, wherein the filaments of the first set of yarns (78) have a thickness in a range of about 4 microns to about 6 microns.

11. The prosthetic valve (10) of claim 5, wherein the filaments of the second set of yarns (80) have a thickness in a range of about 10 microns to about 12 microns.

12. The prosthetic valve (10) of any previous claim, wherein the skirt (16; 200) comprises a satin weave.

13. The prosthetic valve (10) of any previous claim, wherein the leaflet structure (14) comprises a plurality of leaflets (41) having respective inflow edge portions that are sutured to the skirt (16; 200).

14. The prosthetic valve (10) of any previous claim, wherein the skirt (16; 200) is sutured to struts (22, 24, 26, 28, 31, 32, 34) of the frame (12).

## Patentansprüche

1. Implantierbare prothetische Klappe (10), umfassend:
einen ringförmigen Rahmen (12), der ein Einflussende und ein Ausflussende umfasst und radial kollabierbar und expandierbar ist zwischen einer radial kollabierten Konfiguration und einer radial expandierten Konfiguration;
eine Segelstruktur (14), die innerhalb des Rahmens (12) positioniert ist, wobei die Segelstruktur (14) dazu konfiguriert ist, Blutfluss von einem Einflussende zu einem Ausflussende der prothetischen Klappe (10) zu erlauben und den Blutfluss von dem Ausflussende zu einem Einflussende der prothetischen Klappe (10) zu blockieren; und
eine Innenschürze (16; 200) aus Gewebe, die entlang einer Innenoberfläche des Rahmens (12) positioniert ist, wobei die Innenschürze (16; 200) einen ersten Satz von Garnen (78) umfasst, der sich in eine erste Richtung erstreckt, und einen zweiten Satz von Garnen (80), der sich in eine zweite Richtung erstreckt und verwoben ist mit dem ersten Satz von Garnen (78), wobei die Garne des ersten Satzes (78) dünner sind als die Garne des zweiten Satzes (80);
wobei der erste Satz von Garnen (78) verwoben ist mit dem zweiten Satz von Garnen (80), so dass eine Innenoberfläche (202) der Schürze (16; 200) geformt ist aus mehr Garnen des ersten Satzes (78) als Garnen des zweiten Satzes (80) und eine Außenfläche (204) der Schürze (16; 200) geformt ist aus mehr Garnen des zweiten Satzes (80) als Garnen des ersten Satzes (78).

2. Prothetische Klappe (10) gemäß Anspruch 1, wobei die Innenoberfläche (202) der Schürze (16; 200) einen Reibungskoeffizienten aufweist, der geringer ist als ein Reibungskoeffizient der Außenoberfläche (204).

3. Prothetische Klappe (10) gemäß einem der vorangegangenen Ansprüche, wobei die Innenoberfläche (202) der Schürze (16; 200) glatter ist als die Außenoberfläche (204) der Schürze (16; 200).

4. Prothetische Klappe (10) gemäß einem der vorangegangenen Ansprüche, wobei die erste Richtung des ersten Satzes von Garnen (78) sich bezüglich des Rahmens (12) in Umfangsrichtung erstreckt und die zweite Richtung des zweiten Satzes von Garnen (80) sich bezüglich des Rahmens (12) axial erstreckt.

5. Prothetische Klappe (10) gemäß einem der vorangegangenen Ansprüche, wobei die Garne des ersten und des zweiten Satzes von Garnen (78, 80) Multi-Filament-Garne umfasst.

6. Prothetische Klappe (10) gemäß Anspruch 5, wobei die Garne des ersten Satzes (78) eine größere Anzahl von Filamenten als die Garne des zweiten Satzes (80) umfassen.

7. Prothetische Klappe (10) gemäß einem der vorangegangenen Ansprüche, wobei die Schürze (16; 200) ein Gewebe umfasst, das **gekennzeichnet ist durch** mindestens zwei oder mehr benachbarte Garne des ersten Satzes (78), die über einem einzelnen Garn des zweiten Satzes (80) schweben, und mindestens zwei oder mehr Garne des zweiten Satzes (80), die über einem einzelnen Garn des ersten Satzes (78) schweben.

8. Prothetische Klappe (10) gemäß Anspruch 7, wobei die Schürze (16; 200) ein Gewebe umfasst, das **gekennzeichnet ist durch** mindestens vier oder mehr benachbarte Garne des ersten Satzes (78), die über einem einzelnen Garn des zweiten Satzes (80) schweben, und mindestens vier oder mehr Garne des zweiten Satzes (80), die über einem einzelnen Garn des ersten Satzes (78) schweben.

9. Prothetische Klappe (10) gemäß Anspruch 5, wobei die Garne des ersten Satzes (78) ungefähr 30 bis 40 Filamente je Garn aufweisen und die Garne des zweiten Satzes (80) ungefähr 10 bis 20 Filamente je Garn aufweisen.

10. Prothetische Klappe (10) gemäß Anspruch 5, wobei die Filamente des ersten Satzes von Garnen (78) eine Dicke in einem Bereich von ungefähr 4 Mikrometer bis ungefähr 6 Mikrometer aufweisen.

11. Prothetische Klappe (10) gemäß Anspruch 5, wobei die Filamente des zweiten Satzes von Garnen (80) eine Dicke in einem Bereich von ungefähr 10 bis 12 Mikrometer aufweisen.

12. Prothetische Klappe (10) gemäß einem der vorangegangenen Ansprüche, wobei die Schürze (16; 200) ein Satin-Gewebe aufweist.

13. Prothetische Klappe (10) gemäß einem der vorangegangenen Ansprüche, wobei die Segelstruktur (14) eine Vielzahl von Segeln (41) aufweist, die entsprechende Einflusskantenabschnitte aufweisen, die an der Schürze (16; 200) angenäht sind.

14. Prothetische Klappe (10) gemäß einem der vorangegangenen Ansprüche, wobei die Schürze (16; 200) an Streben (22, 24, 26, 28, 31, 32, 34) des Rahmens (12) angenäht ist.

## Revendications

1. Valvule prothétique implantable (10) comprenant :
un cadre annulaire (12) comprenant une extrémité d'entrée et une extrémité de sortie et étant radialement repliable et déployable entre une configuration radialement repliée et une configuration radialement déployée ;
une structure à feuillets (14) positionnée à l'intérieur du cadre (12), la structure à feuillets (14) étant conçue pour permettre l'écoulement du sang depuis une extrémité d'entrée vers une extrémité de sortie de la valvule prothétique (10) et pour bloquer l'écoulement du sang depuis l'extrémité de sortie vers l'extrémité d'entrée de la valvule prothétique (10) ; et
une collerette interne en tissu (16 ; 200) positionnée le long d'une surface interne du cadre (12), la collerette interne (16 ; 200) comprenant un premier ensemble de fils (78) s'étendant dans une première direction et un second ensemble de fils (80) s'étendant dans une seconde direction et tissé avec le premier ensemble de fils (78), les fils du premier ensemble (78) étant plus minces que les fils du second ensemble (80) ;
le premier ensemble de fils (78) étant tissé avec le second ensemble de fils (80) de telle sorte qu'une surface interne (202) de la collerette (16 ; 200) est formée à partir de plus de fils du premier ensemble (78) que de fils du second ensemble (80) et une surface externe (204) de la collerette (16 ; 200) est formée à partir de plus de fils du second ensemble (80) que de fils du premier ensemble (78).

2. Valvule prothétique (10) selon la revendication 1, la surface interne (202) de la collerette (16 ; 200) présentant un coefficient de frottement qui est inférieur à un coefficient de frottement de la surface externe (204).

3. Valvule prothétique (10) selon l'une quelconque revendication précédente, la surface interne (202) de la collerette (16 ; 200) étant plus lisse que la surface externe (204) de la collerette (16 ; 200).

4. Valvule prothétique (10) selon l'une quelconque revendication précédente, la première direction du premier ensemble de fils (78) s'étendant de manière circonférentielle par rapport au cadre (12) et la seconde direction du second ensemble de fils (80) s'étendant axialement par rapport au cadre (12).

5. Valvule prothétique (10) selon l'une quelconque revendication précédente, les fils des premier et second ensembles de fils (78, 80) comprenant des fils multi-filaments.

6. Valvule prothétique (10) selon la revendication 5, les fils du premier ensemble (78) présentant un plus grand nombre de filaments que les fils du second ensemble (80).

7. Valvule prothétique (10) selon l'une quelconque revendication précédente, la collerette (16 ; 200) comprenant une armure **caractérisée par** au moins deux fils adjacents ou plus du premier ensemble (78) flottant sur un seul fil du second ensemble (80) et au moins deux fils ou plus du second ensemble (80) flottant sur un seul fil du premier ensemble (78).

8. Valvule prothétique (10) selon la revendication 7, la collerette (16 ; 200) comprenant une armure **caractérisée par** au moins quatre fils adjacents ou plus du premier ensemble (78) flottant sur un seul fil du second ensemble (80) et au moins quatre fils ou plus du second ensemble (80) flottant sur un seul fil du premier ensemble (78).

9. Valvule prothétique (10) selon la revendication 5, les fils du premier ensemble (78) présentant environ 30 à 40 filaments par fil et les fils du second ensemble (80) présentant environ 10 à 20 filaments par fil.

10. Valvule prothétique (10) selon la revendication 5, les filaments du premier ensemble de fils (78) présentant une épaisseur dans une plage d'environ 4 microns à environ 6 microns.

11. Valvule prothétique (10) selon la revendication 5, les filaments du second ensemble de fils (80) présentant une épaisseur dans une plage d'environ 10 microns à environ 12 microns.

12. Valvule prothétique (10) selon l'une quelconque revendication précédente, la collerette (16 ; 200) comprenant une armure satin.

13. Valvule prothétique (10) selon l'une quelconque revendication précédente, la structure à feuillets (14) comprenant une pluralité de feuillets (41) présentant des parties de bord d'entrée respectives qui sont suturées à la collerette (16 ; 200).

14. Valvule prothétique (10) selon l'une quelconque revendication précédente, la collerette (16 ; 200) étant suturée à des entretoises (22, 24, 26, 28, 31, 32, 34) du cadre (12).
